(19) Europäisches Patentamt
European Patent Office
Office européen des brevets

(11) **EP 3 855 942 B1**

(12) **EUROPEAN PATENT SPECIFICATION**

(45) Date of publication and mention
of the grant of the patent:
**06.04.2022 Bulletin 2022/14**

(21) Application number: **20702129.6**

(22) Date of filing: **03.02.2020**

(51) International Patent Classification (IPC):
*A23L 33/15* (2016.01)     *G16H 20/10* (2018.01)
*G16H 20/60* (2018.01)     *A47J 43/04* (2006.01)
*A47J 43/042* (2006.01)     *G09B 19/00* (2006.01)

(52) Cooperative Patent Classification (CPC):
**A23L 33/15; G09B 19/0092; G16H 20/60;**
A47J 43/042

(86) International application number:
**PCT/EP2020/052527**

(87) International publication number:
**WO 2020/157326 (06.08.2020 Gazette 2020/32)**

(54) **RECOMMENDING INGREDIENTS FOR USE IN THE MAKING OF A NUTRITIONAL PREPARATION**

EMPFEHLUNG VON INHALTSSTOFFEN ZUR VERWENDUNG BEI DER HERSTELLUNG EINER
NAHRUNGSZUBEREITUNG, ENTHALTEND FOLAT

RECOMMANDATION D'INGRÉDIENTS À UTILISER DANS LA FABRICATION D'UNE
PRÉPARATION NUTRITIONNELLE, COMPRENANT DE FOLATE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.02.2019 PCT/CN2019/074692**
**05.08.2019 EP 19190008**

(43) Date of publication of application:
**04.08.2021 Bulletin 2021/31**

(73) Proprietor: **Koninklijke Philips N.V.**
**5656 AG Eindhoven (NL)**

(72) Inventors:
• **ZHAO, Bin**
  **5656 AE Eindhoven (NL)**
• **TANG, Jiani**
  **5656 AE Eindhoven (NL)**
• **LI, Mian**
  **5656 AE Eindhoven (NL)**
• **CHEN, Yun**
  **5656 AE Eindhoven (NL)**
• **BERNARD JOSEPH HIGGINS, Paul**
  **5656 AE Eindhoven (NL)**

(74) Representative: **Kapoor, Pavan Puneet**
**Philips Domestic Appliances Nederland B.V.**
**High Tech Campus 42**
**5656 AE Eindhoven (NL)**

(56) References cited:
**WO-A1-2018/109221    CN-A- 109 456 330**
**JP-A- 2018 002 639    US-A1- 2015 366 405**
**US-A1- 2016 081 515    US-A1- 2018 326 378**

• DELCHIER NICOLAS ET AL: "Thermal
degradation of folates under varying oxygen
conditions", FOOD CHEMISTRY, ELSEVIER LTD,
NL, vol. 165, 23 May 2014 (2014-05-23), pages
85-91, XP029036393, ISSN: 0308-8146, DOI:
10.1016/J.FOODCHEM.2014.05.076
• HANNA SARA STRANDLER ET AL: "Challenges
in the Determination of Unsubstituted Food
Folates: Impact of Stabilities and Conversions on
Analytical Results", JOURNAL OF
AGRICULTURAL AND FOOD CHEMISTRY, vol.
63, no. 9, 26 February 2015 (2015-02-26), pages
2367-2377, XP055665774, US ISSN: 0021-8561,
DOI: 10.1021/jf504987n

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

- **MARGARETHA JÄGERSTAD ET AL:** "Occurrence, Stability, and Determination of Formyl Folates in Foods", JOURNAL OF AGRICULTURAL AND FOOD CHEMISTRY, vol. 61, no. 41, 3 October 2013 (2013-10-03), pages 9758-9768, XP055665778, US ISSN: 0021-8561, DOI: 10.1021/jf4028427
- **M Vignesh ET AL:** "Stabilization of folic acid in liquid dosage form: Formulation development, method validation and comparative analysis", International Journal of Pharmaceutical and Chemical Sciences, 31 March 2012 (2012-03-31), pages 332-338, XP055104243, Retrieved from the Internet: URL:http://ijpcsonline.com/files/39.pdf [retrieved on 2014-02-25]
- **GAZZALI AMIRAH MOHD ET AL:** "Stability of folic acid under several parameters", EUROPEAN JOURNAL OF PHARMACEUTICAL SCIENCES, ELSEVIER, AMSTERDAM, NL, vol. 93, 27 August 2016 (2016-08-27), pages 419-430, XP029736419, ISSN: 0928-0987, DOI: 10.1016/J.EJPS.2016.08.045
- **Anonymous:** "Amazon.com: NutriBullet Balance, Bluetooth Enabled Smart Blender: Kitchen & Dining", , 2 November 2017 (2017-11-02), XP055666248, Retrieved from the Internet: URL:https://www.amazon.com/NutriBullet-Bal ance-Bluetooth-Enabled-Blender/dp/B0773P3W N5?th=1 [retrieved on 2020-02-07]
- **Anonymous:** "Ascent Series A3500 - Smart System Blenders | Vitamix", , 1 January 2017 (2017-01-01), XP055666238, Retrieved from the Internet: URL:https://www.vitamix.com/us/en_us/shop/ a3500 [retrieved on 2020-02-07]

**Description**

FIELD OF THE INVENTION

The invention relates to methods and systems for recommending ingredients for use in the making of a nutritional preparation.

BACKGROUND OF THE INVENTION

Folate, also called "folic acid" or Vitamin B9 is crucial for proper brain function, and plays an important role in mental and emotional health. It also aids in the production of DNA and RNA, and is especially important during infancy, adolescence and pregnancy.

People are able to get folate from food/liquids (nutritional preparations), which contain dietary folate, or nutritional supplements such as vitamin pills, which often contain synthesized folic acid. However, there are some concerns about synthesized folic acid. Unlike natural folates, which are metabolized to tetrahydrofolate (THF) in the mucosa of the small intestine, synthesized folic acid undergoes initial reduction and methylation in the liver, where conversion to the THF form requires dihydrofolate reductase The low activity of this enzyme in the human liver, combined with a high intake of synthesized folic acid, may result in unnatural levels of unmetabolized folic acid entering the systemic circulation.

There is therefore a desire to use dietary folate instead. However, dietary folate has relatively low bioavailability, about 50% of that of pure folic acid. Consequently, there is a desire to improve the bioavailability of dietary folate. The low bioavailability of dietary folate is mainly caused by its low retention during food preparation and cooking.

One way of improving the retention of dietary folate during cooking is to use recipes that are designed for improved retention of dietary folate (e.g. by minimally processing high-folate ingredients).

A recipe defines at least the ingredients to be included in a nutritional preparation. Whilst preferable, a recipe does not need to include instructions on how to make the nutritional preparation. For example, if a recipe is for a blended mixture or smoothie, there is no need to provide instructions as all the ingredients are simply blended together at the same time (which would be appreciated by a user of the recipe).

Methods of automatically identifying recipes are well known in the prior art. By way of example, known methods may identify recipes from a database by processing an input parameters, such as a subject indicating desired ingredients to be used when making a nutritional preparation or indicating a desired nutritional preparation (e.g. a desired meal).

N. Delchier et al. Food chemistry 2014, vol.165, p.85-91 refers to thermal degradation of folates under varying oxygen conditions.

There is an ongoing desire to improve the retention of dietary folate during food preparation and cooking.

SUMMARY OF THE INVENTION

The invention is defined by the claims.

According to examples in accordance with an aspect of the invention, there is provided a computer-implemented method of recommending whether and which one or more additional ingredients should be used by a user in the making of a nutritional preparation to improve folate retention during the making of the nutritional preparation.

The method comprises: receiving a first indicator that indicates whether the user intends to use an oxygen-reduced food processing procedure to make the nutritional preparation; receiving a second indicator that indicates at least one desired ingredient that the user intends to use to make the nutritional preparation, the at least one desired ingredient comprising at least one folate-rich ingredient; determining the pH value of the at least one desired ingredient; and performing a first ingredient recommendation process in response to the first indicator indicating that the user intends to use an oxygen-reduced food processing step during the making of the nutritional preparation.

The first ingredient recommendation process comprises: determining whether the pH value of the at least one desired ingredient is at, above or below a first predetermined pH value; recommending whether and which one or more additional ingredients should be used by: in response to determining that the pH value of the at least one desired ingredient is at or above the first predetermined pH value, recommending that at least one additional ingredient, having a pH value below the first predetermined pH value, is used; and in response to determining that the pH value of the at least one desired ingredient is below the first predetermined pH value, recommending that either no additional ingredient needs to be used or that at least one additional ingredient, having a pH value at or below the pH value of the at least one desired ingredient, is used.

It has been recognized that, if an oxygen-reduced food processing step is used during preparation of the nutritional preparation, then improved folate retention is achieved if the pH value of the combined ingredients used in this step is acidic. It is therefore proposed to identify whether an oxygen-reduced food processing step will be used, and (in response to a positive determination) determine whether additional ingredients are required to bring a pH value down to a suitable acidic level for providing a minimum amount of folate retention.

The invention thereby provides a tool for assisting a user in performing a task of maintaining or improving a folate (folic acid) content of processed food. By including the additional ingredients (if required) in the oxygen-reduced processing step, a folate retention during preparation of the nutritional preparation would be increased or maintained at a minimum level.

The first indicator may be obtained implicitly, e.g. an indicator that a blender or other food processor is set to an oxygen-reduced mode, or explicitly, e.g. via a user input where a user indicates a desired cooking mechanism or processor that uses an oxygen-reduced processed step.

A step of recommending may comprise generating a user-perceptible output, such as a visual, audio and/or haptic output (e.g. via a display, speaker or vibrating element respectively) identifying whether an additional ingredient is recommended and/or the identity of the additional ingredients. In this way, recommending whether and which additional ingredients should be used can assist a user in performing a technical task of improving folate retention during making of a nutritional preparation, and thereby folate content of a nutritional preparation. The improvement to folate retention is directly linked to the recommended ingredients.

The pH value of the at least one desired ingredient may, in particular, be the pH value of the combination of the at least one desired ingredients. This may be calculated by average or appropriately weighted an obtained pH for each desired ingredient.

The first predetermined pH value is no greater than 5.5, and preferably no greater than 5.0. In even more preferable embodiments, the first predetermined pH value is no less than 4.0. By way of example, the first predetermined pH value may in the range of 3.0 to 5.5, or more preferable in the range of 3.0 to 5.0, or more preferably, in the range of 3.0 to 4.0. This increases the folate retention during the making of the nutritional preparation, as increased acidity during an oxygen-reduced food processing step leads to improved folate retention. Preferably, the first predetermined pH value is in the range of 3.0 to 5.0, and preferably in the range of 3.0 to 4.0.

Whilst a low acidity improves the folate retention during making of the nutritional preparation, nutritional preparations having an extremely low acidity may be discomforting or dangerous to consume. Thus, there may be a minimum value for the first predetermined pH value.

In some embodiments, in response to the pH value of the at least one desired ingredient being at or above the first predetermined pH value, the step of recommending may comprise recommending that at least one additional ingredient is used that would bring the combined pH value below the first predetermined value. Similarly, in response to the pH value being below the first predetermined pH value, the step of recommending may comprise recommending that no additional ingredients are required or that ingredients having a pH value below the pH value of the at least one desired ingredient are also used.

In preferred embodiments, the oxygen-reduced food processing step is an oxygen-reduced blending, pureeing, crushing or juicing step.

In at least one embodiment, during the first ingredient recommendation process, the step of recommending whether and which one or more additional ingredients should be used comprises recommending a group of one or more ingredients, including the at least one desired ingredient, that together have a pH value below the first predetermined pH value.

The method may be adapted such that, during the first ingredient recommendation process, the step of recommending whether and which one or more additional ingredients should be used comprises identifying a recipe defining the group of one or more ingredients.

Some methods are adapted wherein, during the first ingredient recommendation process, the step of recommending whether and which one or more additional ingredients should be used comprises: in response to determining that the pH value of the at least one desired ingredient is at or above the first predetermined pH value, providing a user-perceptible output listing a plurality of different additional ingredients having a pH value below the first predetermined pH value, preferably wherein the list is ordered from lowest pH value to highest pH value; and/or in response to determining that the pH value of the at least one desired ingredient is below the first predetermined pH value, providing a user-perceptible output listing a plurality of different additional ingredients having a pH value below the pH value of the at least one desired ingredient, preferably wherein the list is ordered from lowest pH value to highest pH value.

In some embodiments, the second indicator further indicates a weight of each at least one desired ingredient and the method further comprises, after performing the first ingredient recommendation process: receiving a third indicator indicating at least one selected additional ingredient, a selected additional ingredient being one of the plurality of different potential additional ingredients that has been selected by a user; and recommending a minimum weight for each selected additional ingredient so that the combination of the at least one desired ingredient and at least one selected additional ingredient is below the first predetermined pH value.

In some embodiments, the second indicator further indicates a weight of each at least one desired ingredient and the method further comprises, after performing the first ingredient recommendation process and if at least one additional ingredient is recommended, recommending a minimum weight for each at least one additional ingredient so that the combination of the at least one desired ingredient and the at least one additional ingredient is below the first predetermined pH value.

The method further comprise performing a second ingredient recommendation process in response to the first indicator indicating that the user does not intend to use an oxygen-reduced food processing step during the making of the nutritional preparation.

The second ingredient identification process comprises: determining whether the pH value of the at least one desired ingredient is at, above or below a second predetermined pH value; and recommending whether and which one or more additional ingredients should be used by: in response to determining that the pH value of the at least one desired ingredient is at or below the second predetermined pH value, recommending that at least one additional ingredient, having a pH value above the second predetermined pH value, is used; in response to determining that the pH value of the at least one desired ingredient is not below the second predetermined pH value, recommending that no additional ingredient needs to be used or that at least one additional ingredient, having a pH value above the pH value of the at least one desired ingredient, can be used.

It has also been recognized that for a food processing step not performed in a reduced-oxygen environment, then the amount of folate retained during processing decreases with increased acidity. Thus, when there is no oxygen-reduced food processing step, it would be preferable that the folate-rich ingredient is used together with additional ingredients (if required) that are less acidic, e.g. neutral. This improves the folate retention during the making of the nutritional preparation.

The second predetermined pH value is no less than 5.5, preferably no less than 6.0, and more preferably no less than 6.6.

In embodiments, during the second ingredient recommendation process, the step of recommending whether and which one or more additional ingredients should be used comprises: in response to determining that the pH value of the at least one desired ingredient is at or below the second predetermined pH value, providing a user-perceptible output listing a plurality of different additional ingredients having a pH value above the second predetermined pH value, preferably wherein the list is ordered from highest pH value to lowest pH value.

The second indicator may further indicate a weight of each at least one desired ingredient and the method may further comprise, after performing a second ingredient recommendation process in which a user-perceptible output is provided: receiving a fourth indicator indicating at least one selected additional ingredient, a selected additional ingredient being one of the plurality of different potential additional ingredients that has been selected by a user; and recommending a minimum weight for each selected additional ingredient so that the combination of the at least one desired ingredient and at least one selected additional ingredient is above the second predetermined pH value.

According to examples in accordance with an aspect of the invention, there is provided a computer program comprising code means for implementing any previously described when said program is run on a computer.

According to examples in accordance with an aspect of the invention, there is also provided a processing system for recommending whether and which one or more additional ingredients should be used by a user in the making of a nutritional preparation to improve folate retention during the making of the nutritional preparation.

The processing system is adapted to: receive a first indicator that indicates whether the user intends to use an oxygen-reduced food processing procedure to make the nutritional preparation; receive a second indicator that indicates at least one desired ingredient that the user intends to use to make the nutritional preparation, the at least one desired ingredient comprising at least one folate-rich ingredient; determine the pH value of the at least one desired ingredient; and perform a first ingredient recommendation process in response to the first indicator indicating that the user intends to use an oxygen-reduced food processing step during the making of the nutritional preparation

The first ingredient recommendation process comprises: determining whether the pH value of the at least one desired ingredient is at, above or below a first predetermined pH value; recommending whether and which one or more additional ingredients should be used by: in response to determining that the pH value of the at least one desired ingredient is at or above the first predetermined pH value, recommending that at least one additional ingredient, having a pH value below the first predetermined pH value, is used; and in response to determining that the pH value of the at least one desired ingredient is below the first predetermined pH value, recommending that either no additional ingredient needs to be used or that at least one additional ingredient, having a pH value at or below the pH value of the at least one desired ingredient, is used.

According to examples in accordance with an aspect of the invention, there is also provided a method of performing a food processing step during the making of a nutritional preparation using at least one folate-rich ingredient.

The method comprises: grouping the at least one folate-rich ingredient with one or more other ingredients, the grouped ingredients together having an acidic pH value below a first predetermined pH value; and processing the grouped ingredients using an oxygen-reduced food processing step to thereby perform the food processing step.

In preferred embodiments, the first predetermined pH value is no greater than 5.5, and preferably no greater than 5.0.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 is a graph illustrating the effect of acidity on folate retention during an oxygen-reduced food processing step and a non-oxygen recued food processing step;
Figure 2 illustrates a method of recommending a recipe according to an embodiment of the invention;
Figure 3 illustrates a processing system for recommending a recipe according to an embodiment of the invention;
Figure 4 illustrates a method of performing a food processing step according to an embodiment of the invention; and
Figure 5 illustrates an oxygen-reduced food processing unit.

DETAILED DESCRIPTION OF THE EMBODIMENTS

The invention will be described with reference to the Figures.
It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.
The invention provides a method and system for recommending additional ingredients for making a nutritional preparation with improved folate retention. When an oxygen-reduced food processing step is used, folate retention is improved if the ingredients have an acidic pH value. It is proposed to recommend additional or supplementary ingredients to reduce a pH value if a pH value of desired ingredient(s) is above or at a predetermined threshold. There is also proposed a corresponding method for making a nutritional preparation with improved folate retention.
Embodiments of the invention are based on the realization that there is a relationship between folate retention, pH value of ingredients and whether an oxygen-reduced food processing step is used to process the ingredients. In particular, if an oxygen-reduced food processing step is used, folate retention is increased when the ingredients are more acidic. Contrarily, if no oxygen-reduced food processing step is used, folate retention is reduced when the ingredients are more acidic.
Concepts of the invention can be employed in user-friendly software applications (e.g. for a mobile device) or in combination with food processes (e.g. blenders) with an oxygen-reduced food processing option.

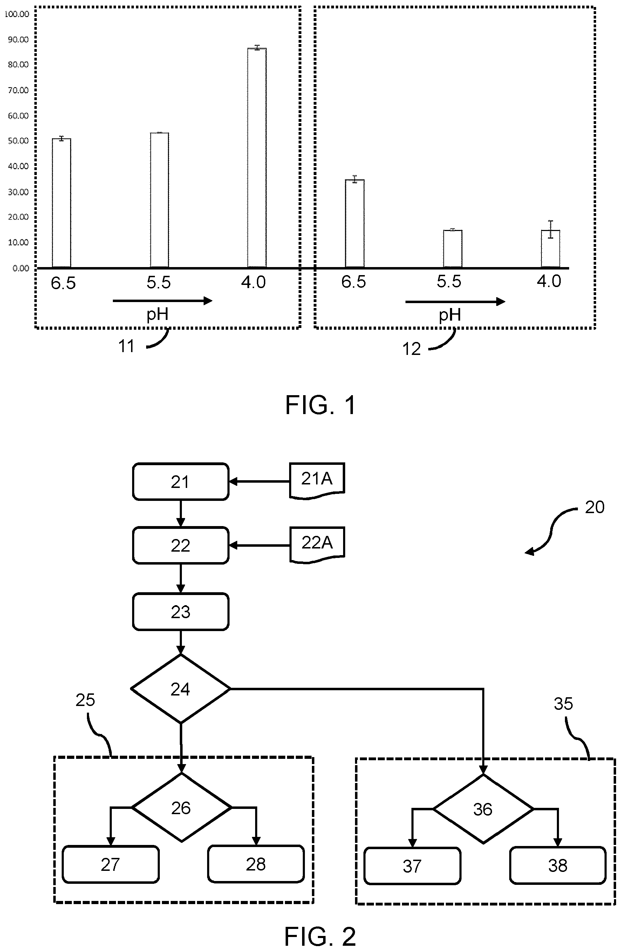

Figure 1 provides two separate graphs for understanding the effect of pH values on folate retention. A first graph 11 illustrates the relationship between pH value (of a group of ingredients) and folate retention when an oxygen-reduced food processing step is used. A second graph 12 illustrates the relationship between pH value (of a group of ingredients) and folate retention when an oxygen-reduced food processing step is not used.
The first graph 11 clearly illustrates how, when an oxygen-reduced food processing step is used, folate retention increases with reduced pH values. Thus, if an oxygen-reduced food processing is used, folate retention can be improved by reducing the pH value (i.e. increasing the acidity) of the ingredients processed.
The second graph 12 illustrates how, when an oxygen-reduced food processing step is not used (i.e. during normal or conventional food processing), folate retention decreases with reduced pH values. Thus, if an oxygen-reduced food processing step is not used, folate retention can be improved by ensuring that the pH value of the ingredients is not acidic.
The data underlying the first 11 and second 12 graphs was obtained using oxygen-reduced blending and conventional (non-oxygen-reduced) blending respectively, although the principles expand to any oxygen-reduced food processing.
Without wishing to be bound by theory, during a food processing step, folate retention is significantly reduced due to leaking of folate due at least to oxidation. Thus, folate retention is improved using oxygen-reduced food processing. It is herein theorized that appropriate food parings could provide a suitable pH value for more L-ascorbic acid retention, which could help stabilize folate. By oxygen-reduced food processing, not only is the stability of folate increased, but so is the protective effect of L-ascorbic acid.
These concepts are employed in the present invention to recommend whether and/or which one or more additional ingredients should be used to improve folate retention during the making of a nutritional preparation. In particular, the pH value of at least one desired ingredients provided by a user is assessed to determine whether an adjustment to the pH value is recommended (i.e. additional ingredients added) in order to improve folate retention or achieve a certain minimum standard of folate retention

Following recommendations thereby enables a user to improve folate retention during making of the nutritional preparation, and thereby improve a folate content of the nutritional preparation. Thus, recommending whether and which additional ingredients should be used credibly assists a user in performing a technical task of improving folate retention during making of a nutritional preparation, and thereby folate content of a nutritional preparation. The improvement to folate retention is directly linked to the recommended ingredients.

Figure 2 illustrates a method 20 according to an embodiment of the invention.

The method 20 comprises a step 21 of receiving a first indicator 21A. The first indicator indicates whether a user intends to use an oxygen-reduced food processing step.

The first indicator may be generated from implicit user actions, e.g. by a user setting a food processor to an oxygen-reduced mode, or explicitly identified by a user, e.g. by a user providing a user input. Other methods of obtaining such a first indicator will be apparent to the skilled person.

The method 20 comprises a step 22 of receiving a second indicator 22A. The second indicator indicates the at least one desired ingredient that the user intends to use to make the nutritional preparation. The at least one desired ingredient includes a folate rich ingredient.

A step 23, of the method 20, comprises determining the pH value of the at least one desired ingredient indicated by the second indicator. Step 23 may comprise, for example, cross-referencing a desired ingredient to a database that links ingredients to pH values.

Table 1 illustrates an extract from a dataset linking potential ingredients to pH values for use in an embodiment of the invention. The skilled person would appreciate that other ingredients may be added to such a dataset (or removed from, e.g. depending upon location, to improve dataset storage efficiency).

Table 1

| Ingredient | pH value |
| --- | --- |
| Lemon | 2.52 |
| Blueberry | 2.64 |
| Kiwi | 3.32 |
| Pineapple | 3.56 |
| Orange | 3.70 |
| Apple | 3.97 |
| Grape | 3.98 |
| Mango | 4.05 |
| Peach | 4.50 |
| Pear | 4.70 |
| Banana | 4.77 |
| Sweet Pepper | 5.04 |
| Watermelon | 5.29 |
| Papaya | 5.38 |
| Beet/Beetroot | 5.75 |
| Cucumber | 5.79 |
| Celery | 5.87 |
| Kale | 6.10 |
| Cabbage | 6.12 |
| Carrot | 6.22 |
| Pumpkin | 6.27 |
| Cantaloupe | 6.34 |
| Spinach | 6.35 |
| Bean Sprouts | 6.38 |
| Red Cabbage | 6.46 |
| Avocado | 6.48 |
| Zucchini | 6.74 |
| Cauliflower | 6.75 |
| Broccoli | 6.77 |

Step 23 may, in particular, comprise determining a combined pH value for the combination of the at least one desired ingredient if there is more than one desired ingredient. In order to calculate the combined pH value, it would be preferable to obtain a weight, volume or other amount-indicating value of each desired ingredient. Preferably, this information is contained in the second indicator or is received from a weighing device, such as a set of scales. Of course, in some embodiments assumptions may be made as to the approximate weight that will be used by the user, e.g. based on historic trends of the user, an average weight of that ingredient used by other users and so on, instead of directly receiving a weight of the desired ingredient. For example, if the lemon juice were indicated as a desired ingredient it can be assumed that the user would use no more than 3 tablespoons of lemon juice based on historic information. Such a step of calculating a combined pH value is not required if only a single desired ingredient is identified.

Moreover, a step of calculating a combined pH value may be omitted by simply taking the pH value of the main or key ingredient. For example, step 23 may comprise identifying the folate rich ingredient (which is likely to be the main ingredient) and determining a pH value of the folate rich ingredient. Thus, the pH value of the folate rich ingredient may represent the pH value of the at least one desired ingredient.

Equation (1) provides one method of accurately calculating a combined pH value pHC for the combination of more than one ingredient (e.g. more than one desired ingredient).

$$pHC = -\log\left(\frac{X1.10^{-pH1} + X2.10^{-pH2} ... + XN.10^{-pHN}}{X1 + X2 ... XN}\right) \tag{1}$$

where XI, X2... XN represents a weight of a respective ingredient and pH1, pH2, ..., pHN represents a pH value of a respective ingredient. Although the above-identified equation for calculating a pH value is designed for liquids or fluids, it has been shown to be sufficiently accurate, for all practical purposes, for use with solids as well.

In some embodiments, e.g. where an amount of a desired ingredient is input (such as in units of tablespoons or cups), a conversion between amount and weight may be automatically performed in order to carry out equation 1 for calculating the combined pH value. This may be performed by, for exampling, referencing a dataset that identifies (for each of a plurality of ingredients) a correspondence between amount and weight of each ingredient, e.g. 1 cup of broccoli florets is approximately 175g in weight or 1 cup of sliced strawberries is approximately 165g in weight.

In response to the first indicator indicating that the user intends to use an oxygen-reduced food processing step, e.g. as determined in a step 24, a first ingredient recommendation process 25 is performed. Thus, step 24 may comprise processing the first indicator to determine whether or not the user intends to use an oxygen-reduced food processing step.

The first ingredient recommendation process 25 recommends whether and which one or more additional ingredients should be used to increase folate retention when an oxygen food processing step is to be used.

In a sub-step 26, the first ingredient recommendation process 25 determines whether the pH value of the at least one desired ingredient (calculated in step 23) is at, above or below a first predetermined pH value. In other words, sub-step 26 comprises determining whether or not the pH value of the at least one desired ingredient is greater than or equal to a first predetermined pH value.

The first predetermined pH value defines whether the at least one desired ingredient is considered sufficiently acidic to provide a suitable level of folate retention. Thus, the first predetermined pH value may vary depending on implementation details. Preferably the first predetermined pH value is no greater than 5.5, and preferably no greater than 5.0, and even more preferably no greater than 4.0. By way of example only, the first predetermined value may be 5.5, 5.0 or 4.0.

Of course, a nutritional preparation having an extremely low acidity may affect a health of the subject, or may taste unpalatable. Thus, in embodiments, the first predetermined pH value is no less than 2.0, for example, no less than 3.0.

In some embodiments, the first predetermined pH value may be a compromise between taste/health and improved folate retention. In preferred embodiments, the first predetermined pH value is in a range from 2.0 to 5.5, for example, from 2.0 to 5.0, for example, from 2.0 to 4.0 or from 2.5 to 5.0 or from 2.5 to 4.0. Other suitable ranges or values for the first predetermined threshold will be apparent to the skilled person.

If the pH value of the at least one desired ingredient is above or at (i.e. greater than or equal to) the first predetermined pH value, than a sub-step 27 is performed. Sub-step 27 comprises recommending that at least one additional ingredient is used, where the at least one additional ingredient has a pH value below the first predetermined value. This has the effect of, when recommendations are followed, reducing the pH value of the ingredients used to make the nutritional preparation. As an oxygen-reduced food processing step is used, this increases folate retention.

In some embodiments, recommending that at least one additional ingredient is used may comprise identifying a selection of different potential additional ingredients, each potential additional ingredient having a pH value below the first predetermined value. This recommendation may be presented in a list format, as set out below.

If the pH value of the least one desired ingredient is below (i.e. less than) the first predetermined pH value, then a sub-step 28 is performed. Sub-step 28 comprises recommending that no additional ingredient is required and/or recommending that at least one additional ingredients can be used, where the additional ingredient has a pH value below that of the pH value of the at least one desired ingredient.

Thus, sub-step 28 may indicate that no additional ingredients are required if the determined pH value of the at least one desired ingredient is below a first predetermined pH value (i.e. as the desired ingredient(s) are already sufficiently acidic to achieve a desired folate retention). However, to further improve folate retention, sub-step 28 may (also or instead) recommend that one or more additional acidic ingredients are added to the desired ingredient to further reduce acidity, and thereby increase folate retention. To ensure that the acidity is further reduced, the at least one additional ingredient should have a pH value below that of the at least one desired ingredients.

For example, sub-step 28 may comprise recommending that no additional ingredients are required to achieve a minimum folate retention, but that one or more additional ingredients having a pH value below that of the at least one desired ingredient could be added to further improve folate retention.

In preferred embodiments, sub-step 27 comprises recommending at least one additional ingredient that, when combined with the at least one desired ingredient, result in a group of ingredients having a pH value below the first predetermined value. This ensures that the combined pH value of the ingredients used in the making of the nutritional preparation are suitable for achieving a minimum level of folate retention.

Of course, sub-step 28 may comprise a similar step of recommending a group of ingredients, including the at least one desired ingredient, having a pH value below the first predetermined value. As no additional ingredient is necessary, this may simply comprise recommending that the desired group of ingredients are used. In other embodiments, this may comprise recommending that further ingredients are used in addition to the desired ingredients to further reduce the pH value of the combined group of ingredients.

Thus, collectively, sub-steps 27 and 28 may be performed in a single step of recommending a group of one or more ingredients, including the at least one desired ingredient, that together have a pH value below the first predetermined value. Alternatively, sub-step 27 and 28 may each be adapted to recommend the group of one or more ingredients.

It has previously been described how the weight(s) of the at least one desired ingredient can be used to calculate the pH value of the at least one desired ingredient. Indeed, the weight(s) of the desired ingredient(s) may be included in the second indicator.

In some further embodiments, in sub-step 27, recommending at least one additional ingredients may comprise identifying a recommended weight for each at least one additional ingredient. The recommended weight(s) may be calculated so as to ensure that the combination of the at least one additional ingredient and the at least one desired ingredient has a total pH value below the first predetermined value.

Equation 2 illustrates one example of formula for determining a weight $X_A$ for an additional ingredient.

$$X_A = X_1 \cdot \frac{10^{-pHT} - 10^{-pHD}}{10^{-pHA} - 10^{-pHT}} \tag{2}$$

where $X_D$ is a (combined) weight of the at least one desired ingredient, pHD is a (combined) pH value of the at least one desired ingredient, pHA is a pH value of the additional ingredient and pHT is a target pH value (e.g. located at or below the first predetermined value).

The target pH value may be equal to the first predetermined value, or may be another predetermined value below the first predetermined value, i.e. less than or equal to the first predetermined value. Equation 2 may be appropriately modified by the skilled person to enable a weight for a plurality of additional ingredients.

In some embodiments, the weight of any given additional ingredient may be limited to a maximum, e.g. depending upon the identity of the ingredient. This may prevent unrealistic ingredient quantities from being recommended (e.g. more than a certain amount of lemon juice). This allows for multiple different additional ingredients to be recommended at a same time.

In some embodiments, sub-step 27 (and optionally sub-step 28) comprises recommending a recipe that defines a group of ingredients including at least the desired ingredient(s). This may comprise, for example, accessing a recipe database containing recipes that define different potential groups of ingredients, and identifying a recipe that defines a group of ingredients having a pH value below the first predetermined value, which group of ingredients includes the at least one desired ingredient.

As previously explained, a recipe defines ingredients to be used in the making of a nutritional preparation. Recipes are typically designed to provide a nutritious, but tasty, nutritional preparation. Thus, by using existing recipes, a likelihood that a group of ingredients that can complement one another in taste is improved. Using existing recipes also improves an ease of recommending additional ingredients.

As illustrated in Figure 2, embodiments may comprise performing a second, different ingredient recommendation process 35 in response to the first indicator indicating that the user does not intend to use an oxygen-reduced food processing step (e.g. as determined in step 24). The second ingredient recommendation process 35 is optional.

The second ingredient recommendation process 35 may comprise a sub-step 36 of determining whether the pH value of the least one ingredient is at, above or below a second predetermined pH value. In other words sub-step 36 comprises determining whether or not the pH value of the at least one desired ingredient is greater than or equal to the second predetermined pH value.

The second predetermined pH value may be different to the first predetermined pH value. This is because folate retention is increased when using an oxygen-reduced food processing method (which appears to be irrespective of acidity level), so that it may not be possible to achieve the same folate retention if not using an oxygen-reduced food processing method.

In response to the pH value of the least one desired ingredient being at or below the second predetermined pH value, a sub-step 37 is performed. Sub-step 37 comprises recommending that at least one additional ingredient, having a pH value above the second predetermined pH value, is used. This has the effect of, by following the recommendation, increasing the pH value of the ingredients used during the making of the food preparation. This increases folate retention.

In response to the pH value of the least one desired ingredient being above the second predetermined pH value, sub-step 38 is performed. Sub-step 38 comprises recommending that no additional ingredient is required, and/or that one or more additional ingredient, having a pH value above the pH value of the least one desired ingredient, can be used. Preferably, the first predetermined pH value is less than (i.e. more acidic than) or equal to the second predetermined pH value. Even more preferably, the first predetermined pH value is less than the second predetermined pH value.

In some embodiments, the second predetermined pH value is no less than 5.5, and preferably no less than 6.0, and more preferably no less than 6.5 or 6.6. The second predetermined pH value may be in the range of from 6.0 to 8.0, for example, from 6.0 to 7.5, for example, from 6.5 to 7.5 or from 6.6 to 7.5 or from 6.5 to 7.2 or from 6.6 to 7.2. For example, the second predetermined pH value may be 7 ($\pm 1.5$ or $\pm 1.0$ or $\pm 0.5$ or $\pm 0.4$), i.e. around neutral.

In any above described embodiment, a step of recommending may comprise generating a user perceptible output that provides information to a user on the recommendation as to whether and which one or more additional ingredients should be used. In particular, a step of recommending may comprise generating a visual, audio and/or haptic output via a respective display, speaker or vibrating element. The generation of the user perceptible output may be integrated into, for example, sub-steps 27 and 28.

When at least one additional ingredient is recommended, the method may comprise recommending one or more potential ingredients that could be used. In particular, the method may comprise consulting a database, which cross-references ingredients to pH values, to identify suitable additional ingredients having a desired pH value.

In particular, examples, when one or more additional ingredients are recommended, a list of potential additional ingredients may be presented to the user. This list may be presented by a user perceptible output device. Each additional ingredient on the list would meet the criteria for an additional ingredient depending upon the embodiment, for example, when an additional ingredient needs to have a pH value below the first predetermined value (i.e. when sub-step 27 is performed) then each additional ingredient on the list would have a pH value below this first predetermined value. This provides a user with a number of options for the additional ingredient to be used.

Preferably, when a list is presented to a subject, the order of additional ingredients on the list is appropriate. In particular, when a list is presented for use during an oxygen-reduced food processing step, the list may be ordered from most acidic to least acidic (i.e. from lowest pH value to highest pH value). When a list is presented for use when no oxygen food processing step is to be used, the list may be ordered from least acidic to most acidic (i.e. from highest pH value to lowest pH value). This increases an ease for the user in understanding appropriate ingredients, and which ones would be most effective in improving folate retention.

The skilled person would be readily capable of adapting the step of recommending whether and which additional ingredients should be used by the user in order to provide a list as explained above.

In some further embodiments, the method further comprises receiving a third indicator indicating at least one selected additional ingredient, a selected additional ingredient being one of the plurality of different potential additional ingredients that has been selected by a user. Thus, a user may select one or more of the listed potential additional ingredients, which selection may be received as a third indicator.

The method may further comprise recommending a minimum weight for each selected additional ingredient so that the combination of the at least one desired ingredient and at least one selected additional ingredient is below the first predetermined pH value (if an oxygen-reduced food processing step is used) or above the second predetermined pH value (if no oxygen-reduced food processing step is used). This may be performed by using equation (2) identified above.

By way of example, consider a first scenario in which an oxygen-reduced food processing step is used, and for which the first predetermined pH value is 5.0 (i.e. this is the target pH value). In this first scenario, the second indicator indicates that the at least one desired ingredient consists of broccoli having a weight of 100g, where it is known, e.g. by referencing a database, that broccoli has a pH value of 6.77. If the user selects "orange" from a presented list of potential additional ingredients, which has a pH value of 3.97, then the minimum weight of orange to be added (to achieve no more than the first predetermined pH value) can be calculated as 10.1g by using equation (2).

By way of another example, consider a second scenario in which an oxygen-reduced food processing step is not used, and for which the second predetermined pH value is 5.5. In this second scenario, the second indicator indicates that the at least one desired ingredient consists of sweet pepper having a weight of 100g, where it is known, e.g. by referencing a database, that sweet pepper has a pH value of 5.04. If the user selects "carrot" from a presented list of potential additional ingredients, which has a pH value of 6.22, then the minimum weight of carrot to be added (to achieve no less than the second predetermined pH value) can be calculated as 232.8g by using equation (2).

To calculate the recommended weight for the at least one additional ingredient, when there is more than one desired ingredient, equation (3) may be used, which is essentially a modified version of equation (2).

$$X_A = X_{D1}.\frac{10^{-pHT} - 10^{-pHD1}}{10^{-pHA} - 10^{-pHT}} + X_{D2}.\frac{10^{-pHT} - 10^{-pHD2}}{10^{-pHA} - 10^{-pHT}} + \cdots \\ + X_{DN}.\frac{10^{-pHT} - 10^{-pHDN}}{10^{-pHA} - 10^{-pHT}} \tag{3}$$

Where $X_A$ is the weight of the selected additional ingredient, pHA is the pH value of the selected additional ingredient, pHT is the target pH value, pHD1, pHD2... pHDN represent pH values of respective desired ingredients and $X_{D1}$, $X_{D2}$ ... $X_{DN}$ represent weights of an Nth desired ingredient.

By way of example, considered a third scenario in which an oxygen-reduced food processing step is used, and for which the first predetermined pH value is 5.0. In this third scenario, the second indicator indicates that the at least one desired ingredient consists of broccoli having a weight of 100g and water having a weight of 100g, where it is known, e.g. by referencing a database, that broccoli has a pH value of 5.04 and water (e.g. in that particular location) has a pH value of 6.87. If the user selects "orange" from a presented list of potential additional ingredients, which has a pH value of 3.97, then the minimum weight of carrot to be added (to achieve no more than the first predetermined pH value) can be calculated as 20.3g by using equation (3).

By way of further example, consider a fourth scenario in which an oxygen-reduced food processing step is not used, and for which the second predetermined pH value is 5.5. In this second scenario, the second indicator indicates that the at least one desired ingredient consists of sweet pepper having a weight of 100g and water having a weight of 100g, where it is known, e.g. by referencing a database, that sweet pepper has a pH value of 5.04 and water (e.g. in that particular location) has a pH value of 6.87. If the user selects "carrot" from a presented list of potential additional ingredients, which has a pH value of 6.22, then the minimum weight of carrot to be added (to achieve no less than the second predetermined pH value) can be calculated as 114.5g by using equation (3).

Of course, the above-identified methods of determining a weight for the at least one additional ingredient may be performed even without the user performing a selection of the at least one additional ingredient, e.g. by presenting a list of potential additional ingredients alongside a weight for each potential additional ingredient to achieve a target pH value, which may be equal/below the first predetermined pH value or equal/above the second predetermined pH value depending on whether the first indicator indicates that the user does or does not intend to use oxygen-reduced food processing step respectively.

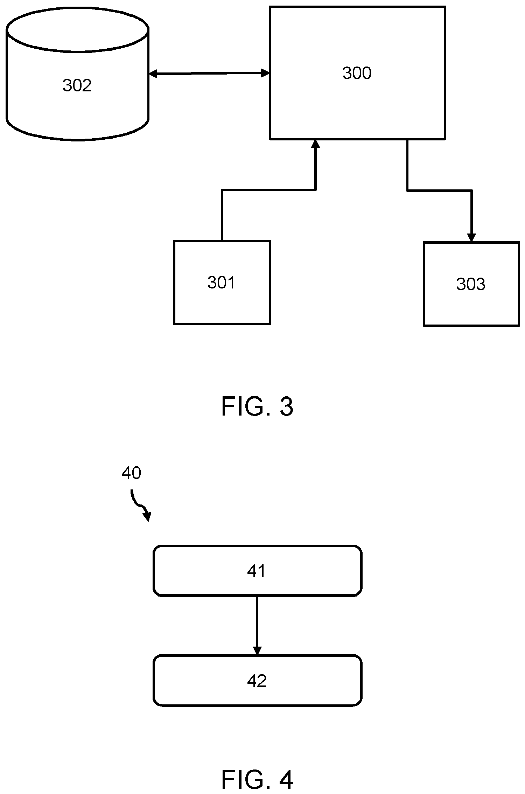

Figure 3 illustrates a processing system 300 for recommending whether and which one or more additional ingredients should be used by a user in the making of a nutritional preparation to improve folate retention during the making of the nutritional preparation.

The processing system 300 is adapted to adapted to receive a first indicator that indicates whether the user intends to use an oxygen-reduced food processing procedure to make the nutritional preparation and receive a second indicator that indicates at least one desired ingredient that the user intends to use to make the nutritional preparation, the at least one desired ingredient comprising at least one folate-rich ingredient.

The processing system 300 may receive the first and second indicators from a user input, e.g. via a user interface 301. In other embodiments, the processing system may receive the first and/or second indicator from food processors or other food-based devices, such as scales. In particular, a food processor operable in an oxygen-reduced food processing mode may provide an indicator to the processing system of whether or not it is in an oxygen-reduced food processing mode to thereby provide the first indicator.

The processing system 300 is adapted to determine the pH value of the at least one desired ingredient. This may comprise referencing a database or memory 302 (e.g. a cloud memory) to identify a pH value of the at least one desired ingredient.

The processing system is further adapted to perform a first ingredient recommendation process in response to the first indicator indicating that the user intends to use an oxygen-reduced food processing step during the making of the nutritional preparation. The first ingredient recommendation process comprises: determining whether the pH value of the at least one desired ingredient is at, above or below a first predetermined pH value; recommending whether and which one or more additional ingredients should be used by: in response to determining that the pH value of the at least one desired ingredient is at or above the first predetermined pH value, recommending that at least one additional ingredient, having a pH value below the first predetermined pH value, is used; and in response to determining that the pH value of the at least one desired ingredient is below the first predetermined pH value, recommending that either no additional ingredient needs to be used or that at least one additional ingredient, having a pH value at or below the pH value of the at least one desired ingredient, is used.

The processing system 300 may be adapted to communicate with an output user interface, such as a display 303, to provide the recommendations to a user.

Of course, the skilled person will appreciate that the processing system 300 may be adapted to carry out any previously described method.

Figure 4 illustrates a method 40 according to another embodiment of the invention. The method 40 exploits the recognition that an acidic pH value improves folate retention in oxygen-reduced food processing. Method 40 relates to a food processing step for use during the making of a nutritional preparation using at least one folate-rich ingredient.

The method 40 comprises a step 41 of grouping the at least one folate-rich ingredient with one or more other ingredients, the grouped ingredients together having an acidic pH value below a first predetermined pH value.

The method then comprises a step 42 of processing the grouped ingredients using an oxygen-reduced food processing step to thereby perform the food processing step.

The first predetermined pH value defines whether the grouped ingredients are sufficiently acidic to provide a suitable or desired level of folate retention. Thus, the first predetermined pH value may vary depending on implementation details. Preferably, the first predetermined pH value is no greater than 5.5, and preferably no greater than 5.0, and even more preferably no greater than 4.0. By way of example, only, the first predetermined value may be 5.5, 5.0 or 4.0.

Of course, a nutritional preparation having an extremely low acidity may affect a health of the subject, or may taste unpalatable. Thus, in embodiments, the first predetermined pH value is no less than 2.0, for example, no less than 3.0. In some embodiments, the first predetermined pH value may be a compromise between taste/health and improved folate retention. In preferred embodiments, the first predetermined pH value is in a range from 2.0 to 5.5, for example, from 2.0 to 5.0, for example, from 2.0 to 4.0 or from 2.5 to 5.0 or from 2.5 to 4.0. Other suitable ranges or values for the first predetermined threshold will be apparent to the skilled person.

Embodiments may be appropriately adapted based on other concepts proposed in the present application. For example, the step 41 of grouping the ingredients may comprise identifying one or more additional ingredients to accompany the at least one folate-rich ingredient using any above-identified recommendation process. In another example, step 41 may comprise determining a weight of the one or more other ingredients based upon the weight(s) of the least one folate-rich ingredient, e.g. using equation 2 as a basis.

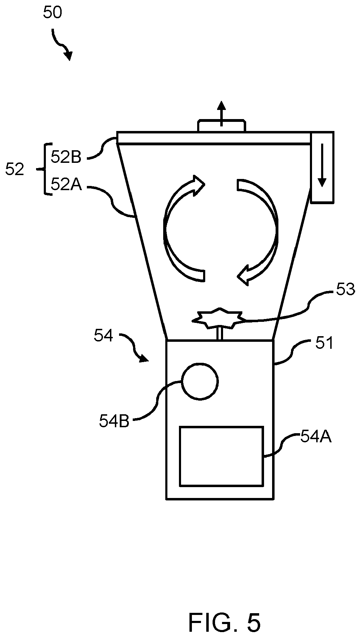

Figure 5 illustrates an oxygen-reduced food processor, in particular a blender 50, according to an embodiment of the invention.

The blender 50 comprises a base 51 and a (removable) food container 52. The removable food container 52 may comprise a housing 52A and a lid 52B, which may be detachable from one another

One or more blades 53 is connected to the base and extends into the food container. The one or more blades 53 are adapted to (selectively/controllably) rotate so as to chop or slice ingredients contained in the food container (when the food container is connected to the base 51), thereby blending the ingredients. Blending is indicated by appropriate curved arrows. The speed, pulse pattern and/or activation (i.e. ON or OFF) of the blades 53 can be controlled via a user interface 54. The user interface may comprise a (touch-sensitive) display 54A and/or a dial 54B (e.g. which may define the speed of the blades).

The blender 50 is adapted to extract air from the blender (specifically, from the food container), e.g. using a pump, as indicated by the straight arrows. Alternatively, the blender 50 may replace the air within the food container with a gas that is not oxygen, e.g. nitrogen or a noble gas. This reduces an oxygen content in the blender. Preferably, the blender extracts the air from the blender prior to blending ingredients within the blender, i.e. before the blades begin rotating, but may also begin extracting the air during a blending process. In this way, the blender is adapted to blend or process received ingredients in an oxygen-reduced environment.

Of course, the blender may be operable in both an oxygen-reduced mode (where oxygen is removed or replaced, as previously described) and a "normal" processing mode (in which oxygen is not removed or replaced). Control over which mode the blender operates may be controlled by the user, e.g. via the user interface 54.

The blender 50 may also comprise the processing system (not shown) previously described. In embodiments, the display 54A is adapted to display the recommendation obtained by the processing system, e.g. whether and which additional ingredients should be used. The first indicator may be received by determining a mode of the blender, and the second indicator may be received via a user input, e.g. via the user interface.

In other embodiments, the blender is adapted to communicate with a separate processing system (e.g. located in a cloud-computing environment). For example, the blender may pass a first and second indicator to the processing system and receive a recommendation from the processing system (which may be displayed on the display 54A). In embodiments, the blender is adapted to communicate with a handheld device of the user, such as a mobile phone, which may allow them to at least input their desired ingredients and/or act as the processing system.

In some embodiments, the blender may comprise an inbuilt set of scales for measuring the weight of ingredients placed in the food container. This allows for automatic calculation of the weight of the desired ingredients (as they have been placed in the food container). This may allow for increased ease in determining the combined pH value of the desired ingredients.

In conceivable embodiments, the blender may comprise a food identification unit, e.g. a camera and an image processor (e.g. integrated into the processing system). Methods of automatically identifying food would be apparent to the skilled person. The food identification unit may identify food placed in the blender (and optionally a weight) to thereby determine the second indicator indicating at least one desired ingredient.

Concepts described for the blender are extendible to juicers, pureeing system or crushing systems, e.g. by appropriate replacement of the blades with other mechanisms. Inventive concepts are particularly advantageous when used to recommend ingredients when an oxygen-reduced blending, puréeing, crushing or juicing step is performed. However, other oxygen-reduced food processing steps are also envisaged, such as low oxidative cooking. In preferred embodiments, the oxygen-reduced food processing step is a vacuum food processing step.

Where reference is made to the pH value of the at least one desired ingredient, this may refer to the combined pH value of the combination of the at least one desired ingredient. The term "ingredient" can refer to either the merely the identity of product usable in making a nutritional preparation or to the weight and identity of the product usable in making a nutritional preparation. As is known in the art, the term "acidic" refers to a substance or mixture having a pH value less than 7.0. The term "nutritional preparation" refers to any food or liquid preparation made from nutritional ingredients, such as fruit, vegetables, meat, dairy products etc. Examples of nutritional preparations include meals, smoothies, food product etc A nutritional preparation may form part of a meal, e.g. a sauce for the meal.

The meaning of a "folate-rich ingredient" or "high-folate ingredient" would be readily apparent to the skilled person, meaning an ingredient that has a relatively high amount of folate per weight. By way of example only, a "folate-rich ingredient" may mean any ingredient having a folate concentration no less than $10\mu g$ per 100g weight of the ingredient, for example a folate concentration no less than $15\mu g$ or $20\mu g$ per 100g weight. Table 2 illustrates examples of folate rich ingredients along with their folate concentrations, as taken from the USDA Food Composition Database (which the skilled person would consider using to identify other folate rich ingredients).

Table 2

| Ingredient | Folate Concentration ($\mu$g/100g) |
|---|---|
| **Vegetable** | |
| Spinach | 194 |
| Brussels Sprouts | 61 |
| Broccoli | 63 |
| Asparagus | 52 |
| Turnip | 15 |
| Collard Greens | 129 |
| Okra | 60 |
| **Fruit** | |
| Strawberry | 24 |
| Orange | 30 |
| Cantaloupe | 21 |
| **Legumes** | |
| Peanut | 240 |
| Pinto Bean | 525 |
| Black Bean | 444 |
| Lima Bean | 395 |
| Kidney Bean | 394 |
| Lentil | 479 |

According to a concept, there is proposed a method of recommending a group ingredients, including at least one folate-rich ingredient, to be used by user to make a nutritional preparation. The method comprises receiving an indicator of whether the user intends to use an oxygen-reduced food processing step during the making of the nutritional preparation; and in response to the indicator indicating that the user intends to use an oxygen-reduced food processing step during the making of the nutritional preparation, identifying a group of ingredients that together have an acidic pH value below a first predetermined pH value and include a folate-rich ingredient.

The group of ingredients may include one or more desired ingredients identified by a subject, so that the step of identifying a group of ingredients comprises identifying one or more additional ingredients that bring the pH value of the group of ingredients below the first predetermined pH value.

The skilled person would be readily capable of developing a processing system for carrying out herein described methods. Thus, each step of the flow chart may represent a different action performed by a processing system, and may be performed by a respective module of the processing system.

Embodiments may therefore make use of a processing system, e.g. as previously described. The processing system can be implemented in numerous ways, with software and/or hardware, to perform the various functions required. A processor is one example of a processing system, which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

**Claims**

1. A computer-implemented method of recommending whether and which one or more additional ingredients should be used by a user in the making of a nutritional preparation to improve folate retention during the making of the nutritional preparation, the method comprising:

   receiving a first indicator that indicates whether the user intends to use an oxygen-reduced food processing procedure to make the nutritional preparation;
   receiving a second indicator that indicates at least one desired ingredient that the user intends to use to make the nutritional preparation, the at least one desired ingredient comprising at least one folate-rich ingredient;
   determining the pH value of the at least one desired ingredient; and
   performing a first ingredient recommendation process in response to the first indicator indicating that the user intends to use an oxygen-reduced food processing step during the making of the nutritional preparation, wherein the first ingredient recommendation process comprises:

   determining whether the pH value of the at least one desired ingredient is at, above or below a first predetermined pH value, wherein the first predetermined pH value is no greater than 5.5;
   recommending whether and which one or more additional ingredients should be used by:

   in response to determining that the pH value of the at least one desired ingredient is at or above the first predetermined pH value, recommending that at least one additional ingredient, having a pH value below the first predetermined pH value, is used; and
   in response to determining that the pH value of the at least one desired ingredient is below the first predetermined pH value, recommending that either no additional ingredient needs to be used or that at least one additional ingredient, having a pH value at or below the pH value of the at least one desired ingredient, is used;

   or

performing a second ingredient recommendation process in response to the first indicator indicating that the user does not intend to use an oxygen-reduced food processing step during the making of the nutritional preparation, the second ingredient identification process comprising:

determining whether the pH value of the at least one desired ingredient is at, above or below a second predetermined pH value, wherein the second predetermined pH value is no less than 5.5; and
recommending whether and which one or more additional ingredients should be used by:

in response to determining that the pH value of the at least one desired ingredient is at or below the second predetermined pH value, recommending that at least one additional ingredient, having a pH value above the second predetermined pH value, is used;
in response to determining that the pH value of the at least one desired ingredient is not below the second predetermined pH value, recommending that no additional ingredient needs to be used or that at least one additional ingredient, having a pH value above the pH value of the at least one desired ingredient, can be used.

2. The method of claim 1, wherein the first predetermined pH value is no greater than 5.0, preferably in the range of from 3.0 to 5.0, and more preferably in the range of from 3.0 to 4.0.

3. The method of any of claims 1 or 2, wherein the oxygen-reduced food processing step is an oxygen-reduced blending, pureeing, crushing or juicing step.

4. The method of any of claims 1 to 3, wherein, during the first ingredient recommendation process, the step of recommending whether and which one or more additional ingredients should be used comprises recommending a group of one or more ingredients, including the at least one desired ingredient, that together have a pH value below the first predetermined pH value.

5. The method of claim 4, wherein, during the first ingredient recommendation process, the step of recommending whether and which one or more additional ingredients should be used comprises identifying a recipe defining the group of one or more ingredients.

6. The method of any of claims 1 to 5, wherein, during the first ingredient recommendation process, the step of recommending whether and which one or more additional ingredients should be used comprises:

in response to determining that the pH value of the at least one desired ingredient is at or above the first predetermined pH value, providing a user-perceptible output listing a plurality of different additional ingredients having a pH value below the first predetermined pH value, preferably wherein the list is ordered from lowest pH value to highest pH value; and/or
in response to determining that the pH value of the at least one desired ingredient is below the first predetermined pH value, providing a user-perceptible output listing a plurality of different additional ingredients having a pH value below the pH value of the at least one desired ingredient, preferably wherein the list is ordered from lowest pH value to highest pH value.

7. The method of claim 6, wherein the second indicator further indicates a weight of each at least one desired ingredient and the method further comprises, after performing the first ingredient recommendation process:

receiving a third indicator indicating at least one selected additional ingredient, a selected additional ingredient being one of the plurality of different potential additional ingredients that has been selected by a user; and
recommending a minimum weight for each selected additional ingredient so that the combination of the at least one desired ingredient and at least one selected additional ingredient is below the first predetermined pH value.

8. The method of any of claims 1 to 7, wherein the second indicator further indicates a weight of each at least one desired ingredient and the method further comprises, after performing the first ingredient recommendation process and if at least one additional ingredient is recommended, recommending a minimum weight for each at least one additional ingredient so that the combination of the at least one desired ingredient and the at least one additional ingredient is below the first predetermined pH value.

9. The method of claim 1, wherein the second predetermined pH value is no less than 6.0, and more preferably no less than 6.6.

10. The method of any of claims 1 or 9, wherein, during the second ingredient recommendation process, the step of recommending whether and which one or more additional ingredients should be used comprises:
in response to determining that the pH value of the at least one desired ingredient is at or below the second predetermined pH value, providing a user-perceptible output listing a plurality of different additional ingredients having a pH value above the second predetermined pH value, preferably wherein the list is ordered from highest pH value to lowest pH value.

11. The method of claim 10, wherein the second indicator further indicates a weight of each at least one desired ingredient and the method further comprises, after performing a second ingredient recommendation process in which a user-perceptible output is provided:

receiving a fourth indicator indicating at least one selected additional ingredient, a selected additional ingredient being one of the plurality of different potential additional ingredients that has been selected by a user; and recommending a minimum weight for each selected additional ingredient so that the combination of the at least one desired ingredient and at least one selected additional ingredient is above the second predetermined pH value.

12. A computer program comprising code means for implementing the method of any one of claims 1 to 11 when said program is run on a computer.

13. A processing system for determining whether any additional ingredients need to be used by a user to ensure at least a predetermined amount of folate retention in the making of a nutritional preparation, the processing system being adapted to:

receive a first indicator that indicates whether the user intends to use an oxygen-reduced food processing step during the making of the nutritional preparation;
receive a second indicator that indicates at least one desired ingredient that the user intends to use in the making of the nutritional preparation;
determine the pH value of the at least one desired ingredient; and
perform a first ingredient recommendation process in response to the first indicator indicating that the user does intend to use an oxygen-reduced food processing step during the making of the nutritional preparation, the first ingredient recommendation process comprising:

determining whether the pH value of the at least one desired ingredient is at, above or below a first predetermined pH value, wherein the first predetermined pH value is no greater than 5.5;
recommending whether and which one or more additional ingredients should be used by:

in response to determining that the pH value of the at least one desired ingredient is at or above the first predetermined pH value, recommending that at least one additional ingredient, having a pH value below the first predetermined pH value, is used; and
in response to determining that the pH value of the at least one desired ingredient is below the first predetermined pH value, recommending that either no additional ingredient needs to be used or that at least one additional ingredient, having a pH value at or below the pH value of the at least one desired ingredient, is used;

or

performing a second ingredient recommendation process in response to the first indicator indicating that the user does not intend to use an oxygen-reduced food processing step during the making of the nutritional preparation, the second ingredient identification process comprising:

determining whether the pH value of the at least one desired ingredient is at, above or below a second predetermined pH value, wherein the second predetermined pH value is no less than 5.5; and
recommending whether and which one or more additional ingredients should be used by:

in response to determining that the pH value of the at least one desired ingredient is at or below the second predetermined pH value, recommending that at least one additional ingredient, having a pH value above the second predetermined pH value, is used;

in response to determining that the pH value of the at least one desired ingredient is not below the second predetermined pH value, recommending that no additional ingredient needs to be used or that at least one additional ingredient, having a pH value above the pH value of the at least one desired ingredient, can be used.

14. The processing system of claim 13, wherein the first predetermined pH value is no greater than 5.0, wherein the first predetermined pH value is preferably in the range of from 3.0 to 5.0, and more preferably in the range of from 3.0 to 4.0; and wherein the second predetermined pH value is no less than 6.0.


**Patentansprüche**

1. Computerimplementiertes Verfahren zur Empfehlung, ob und welche von einem oder mehreren zusätzlichen Inhaltstoffen von einem Benutzer bei der Herstellung einer Nahrungszubereitung verwendet werden sollten, um die Folateretention während der Herstellung der Nahrungszubereitung zu verbessern, wobei das Verfahren umfasst:

Empfangen eines ersten Indikators, der anzeigt, ob der Benutzer beabsichtigt, ein sauerstoffreduziertes Lebensmittelverarbeitungsverfahren zu verwenden, um die Nahrungszubereitung herzustellen;
Empfangen eines zweiten Indikators, der mindestens einen gewünschten Inhaltsstoff anzeigt, den der Benutzer zu verwenden beabsichtigt, um die Nahrungszubereitung herzustellen, wobei der mindestens eine gewünschte Inhaltsstoff mindestens einen folatreichen Inhaltsstoff umfasst;
Bestimmen des pH-Wertes des mindestens einen gewünschten Inhaltsstoffs; und
Durchführen eines ersten Inhaltsstoffempfehlungsprozesses als Reaktion darauf, dass der erste Indikator anzeigt, dass der Benutzer beabsichtigt, einen sauerstoffreduzierten Lebensmittelverarbeitungsschritt während der Herstellung der Nahrungszubereitung zu verwenden, wobei der erste Inhaltstoffempfehlungsprozess umfasst:

Bestimmen, ob der pH-Wert des mindestens einen gewünschten Inhaltsstoffs bei, über oder unter einem ersten vorbestimmten pH-Wert liegt, wobei der erste vorbestimmte pH-Wert nicht größer als 5,5 ist;
Empfehlen, ob und welche von einem oder mehreren zusätzlichen Inhaltsstoffe verwendet werden sollten durch:

als Reaktion auf die Feststellung, dass der pH-Wert des mindestens einen gewünschten Inhaltsstoffs bei oder über dem ersten vorbestimmten pH-Wert liegt, Empfehlen, dass mindestens ein zusätzlicher Inhaltsstoff mit einem pH-Wert unter dem ersten vorbestimmten pH-Wert verwendet wird; und
als Reaktion auf die Feststellung, dass der pH-Wert des mindestens einen gewünschten Inhaltsstoffs unter dem ersten vorbestimmten pH-Wert liegt, Empfehlen, dass entweder kein zusätzlicher Inhaltsstoff verwendet werden muss oder dass mindestens ein zusätzlicher Inhaltsstoff mit einem pH-Wert bei oder unter dem pH-Wert des mindestens einen gewünschten Inhaltsstoffs verwendet wird;

oder

Durchführen eines zweiten Inhaltstoffempfehlungsprozesses als Reaktion darauf, dass der erste Indikator anzeigt, dass der Benutzer nicht beabsichtigt, einen sauerstoffreduzierten Lebensmittelverarbeitungsschritt während der Herstellung der Nahrungszubereitung zu verwenden, wobei der zweite Inhaltstoffidentifizierungsprozess umfasst:

Bestimmen, ob der pH-Wert des mindestens einen gewünschten Inhaltsstoffs bei, über oder unter einem zweiten vorbestimmten pH-Wert liegt, wobei der zweite vorbestimmte pH-Wert nicht weniger als 5,5 ist; und
Empfehlen, ob und welche von einem oder mehreren zusätzlichen Inhaltsstoffe verwendet werden sollten durch:

als Reaktion auf die Feststellung, dass der pH-Wert des mindestens einen gewünschten Inhaltsstoffs bei oder unter dem zweiten vorbestimmten pH-Wert liegt, Empfehlen, dass mindestens ein zusätzlicher Inhaltsstoff mit einem pH-Wert über dem zweiten vorbestimmten pH-Wert verwendet wird;
als Reaktion auf die Feststellung, dass der pH-Wert des mindestens einen gewünschten Inhaltsstoffs nicht unter dem zweiten vorbestimmten pH-Wert liegt, Empfehlen, dass kein zusätzlicher Inhaltsstoff verwendet werden muss oder dass mindestens ein zusätzlicher Inhaltsstoff mit einem pH-Wert über dem pH-Wert des mindestens einen gewünschten Inhaltsstoffs verwendet werden kann.

**2.** Verfahren nach Anspruch 1, wobei der erste vorbestimmte pH-Wert nicht größer als 5,0, vorzugsweise im Bereich von 3,0 bis 5,0 und besonders bevorzugt im Bereich von 3,0 bis 4,0 liegt.

**3.** Verfahren nach einem der Ansprüche 1 oder 2, wobei der sauerstoffreduzierte Lebensmittelverarbeitungsschritt ein sauerstoffreduzierter Misch-, Pürier-, Zerkleinerungsoder Entsaftungsschritt ist.

**4.** Verfahren nach einem der Ansprüche 1 bis 3, wobei während des ersten Inhaltsstoffempfehlungsprozesses der Schritt des Empfehlens, ob und welche von einem oder mehreren zusätzlichen Inhaltsstoffe verwendet werden sollten, das Empfehlen einer Gruppe von einem oder mehreren Inhaltsstoffen umfasst, einschließlich der mindestens einen gewünschten Inhaltsstoff, die zusammen einen pH-Wert unter dem ersten vorbestimmten pH-Wert aufweisen.

**5.** Verfahren nach Anspruch 4, wobei während des ersten Inhaltstoffempfehlungsprozesses der Schritt des Empfehlens, ob und welche von einem oder mehreren zusätzlichen Inhaltsstoffe verwendet werden sollten, das Identifizieren eines Rezepts umfasst, das die Gruppe von einem oder mehreren Inhaltsstoffen definiert.

**6.** Verfahren nach einem der Ansprüche 1 bis 5, wobei während des ersten Inhaltsstoffempfehlungsprozesses der Schritt des Empfehlens, ob und welche von einem oder mehreren zusätzlichen Inhaltsstoffe verwendet werden sollten, umfasst:

als Reaktion auf die Feststellung, dass der pH-Wert des mindestens einen gewünschten Inhaltsstoffs bei oder über dem ersten vorbestimmten pH-Wert liegt, Bereitstellen einer für den Benutzer wahrnehmbaren Ausgabe, die eine Vielzahl von verschiedenen zusätzlichen Inhaltsstoffen mit einem pH-Wert unter dem ersten vorbestimmten pH-Wert auflistet, vorzugsweise wobei die Liste vom niedrigsten pH-Wert zum höchsten pH-Wert geordnet ist; und/oder
als Reaktion auf die Feststellung, dass der pH-Wert des mindestens einen gewünschten Inhaltsstoffs unter dem ersten vorbestimmten pH-Wert liegt, Bereitstellen einer für den Benutzer wahrnehmbaren Ausgabe, die eine Vielzahl von verschiedenen zusätzlichen Inhaltsstoffen mit einem pH-Wert unter dem pH-Wert des mindestens einen gewünschten Inhaltsstoffs auflistet, wobei die Liste vorzugsweise vom niedrigsten pH-Wert zum höchsten pH-Wert geordnet ist.

**7.** Verfahren nach Anspruch 6, wobei der zweite Indikator ferner ein Gewicht jedes mindestens einen gewünschten Inhaltsstoffs anzeigt und das Verfahren ferner umfasst, nach dem Durchführen des ersten Inhaltsstoffempfehlungsprozesses:

Empfangen eines dritten Indikators, der mindestens einen ausgewählten zusätzlichen Inhaltsstoff anzeigt, wobei ein ausgewählter zusätzlicher Inhaltsstoff einer der Vielzahl von verschiedenen potentiellen zusätzlichen Inhaltsstoffen ist, der von einem Benutzer ausgewählt wurde; und
Empfehlen eines Mindestgewichts für jeden ausgewählten zusätzlichen Inhaltsstoff, so dass die Kombination des zumindest einen gewünschten Inhaltsstoffs und zumindest eines ausgewählten zusätzlichen Inhaltsstoffs unter dem ersten vorbestimmten pH-Wert liegt.

**8.** Verfahren nach einem der Ansprüche 1 bis 7, wobei der zweite Indikator ferner ein Gewicht jedes mindestens einen gewünschten Inhaltsstoffs anzeigt und das Verfahren ferner umfasst, nach dem Durchführen des ersten Inhaltsstoffempfehlungsprozesses und wenn mindestens ein zusätzlicher Inhaltsstoff empfohlen wird, Empfehlen eines Mindestgewichts für jeden mindestens einen zusätzlichen Inhaltsstoff, so dass die Kombination des mindestens einen gewünschten Inhaltsstoffs und des mindestens einen zusätzlichen Inhaltsstoffs unter dem ersten vorbestimmten pH-Wert liegt.

**9.** Verfahren nach Anspruch 1, wobei der zweite vorbestimmte pH-Wert nicht weniger als 6,0 und besonders bevorzugt nicht weniger als 6,6 liegt.

**10.** Verfahren nach einem der Ansprüche 1 oder 9, wobei während des zweiten Inhaltsstoffempfehlungsprozesses der Schritt des Empfehlens, ob und welche von einem oder mehreren zusätzlichen Inhaltsstoffe verwendet werden sollten, umfasst:

als Reaktion auf die Feststellung, dass der pH-Wert des mindestens einen gewünschten Inhaltsstoffs bei oder unter dem zweiten vorbestimmten pH-Wert liegt, Bereitstellen einer vom Benutzer wahrnehmbaren Ausgabe, die eine Vielzahl von verschiedenen zusätzlichen Inhaltsstoffen mit einem pH-Wert über dem zweiten vorbestimmten pH-Wert auflistet, wobei die Liste vorzugsweise vom höchsten pH-Wert zum niedrigsten pH-Wert geordnet ist.

11. Verfahren nach Anspruch 10, wobei der zweite Indikator ferner ein Gewicht jedes mindestens einen gewünschten Inhaltsstoffs anzeigt und das Verfahren ferner umfasst, nachdem ein zweiter Inhaltsstoffempfehlungsprozess durchgeführt wurde, bei dem eine vom Benutzer wahrnehmbare Ausgabe bereitgestellt wird:

Empfangen eines vierten Indikators, der mindestens einen ausgewählten zusätzlichen Inhaltsstoff anzeigt, wobei ein ausgewählter zusätzlicher Inhaltsstoff einer der Vielzahl von unterschiedlichen potentiellen zusätzlichen Inhaltsstoffen ist, der von einem Benutzer ausgewählt wurde; und
Empfehlen eines Mindestgewichts für jeden ausgewählten zusätzlichen Inhaltsstoff, so dass die Kombination des zumindest einen gewünschten Inhaltsstoffs und zumindest eines ausgewählten zusätzlichen Inhaltsstoffs über dem zweiten vorbestimmten pH-Wert liegt.

12. Computerprogramm mit Codemitteln zum Implementieren des Verfahrens nach einem der Ansprüche 1 bis 11, wenn das Programm auf einem Computer ausgeführt wird.

13. Verarbeitungssystem zum Bestimmen, ob zusätzliche Inhaltsstoffe von einem Benutzer verwendet werden müssen, um mindestens eine vorbestimmte Menge von Folateretention bei der Herstellung einer Nahrungszubereitung sicherzustellen, wobei das Verarbeitungssystem angepasst ist zum:

Empfangen eines ersten Indikators, der anzeigt, ob der Benutzer beabsichtigt, einen sauerstoffreduzierten Lebensmittelverarbeitungsschritt während der Herstellung der Nahrungszubereitung zu verwenden;
Empfangen eines zweiten Indikators, der mindestens einen gewünschten Inhaltsstoff anzeigt, den der Benutzer bei der Herstellung der Nahrungszubereitung zu verwenden beabsichtigt;
Bestimmen des pH-Wertes des mindestens einen gewünschten Inhaltsstoffs; und
Durchführen eines ersten Inhaltstoffempfehlungsprozesses als Reaktion darauf, dass der erste Indikator anzeigt, dass der Benutzer beabsichtigt, einen sauerstoffreduzierten Lebensmittelverarbeitungsschritt während der Herstellung der Nahrungszubereitung zu verwenden, wobei der erste Inhaltstoffempfehlungsprozess umfasst:

Bestimmen, ob der pH-Wert des mindestens einen gewünschten Inhaltsstoffs bei, über oder unter einem ersten vorbestimmten pH-Wert liegt, wobei der erste vorbestimmte pH-Wert nicht größer als 5,5 ist;
Empfehlen, ob und welche von einem oder mehreren zusätzlichen Inhaltsstoffe verwendet werden sollten durch:

als Reaktion auf die Feststellung, dass der pH-Wert des mindestens einen gewünschten Inhaltsstoffs bei oder über dem ersten vorbestimmten pH-Wert liegt, Empfehlen, dass mindestens ein zusätzlicher Inhaltsstoff mit einem pH-Wert unter dem ersten vorbestimmten pH-Wert verwendet wird; und
als Reaktion auf die Feststellung, dass der pH-Wert des mindestens einen gewünschten Inhaltsstoffs unter dem ersten vorbestimmten pH-Wert liegt, Empfehlen, dass entweder kein zusätzlicher Inhaltsstoff verwendet werden muss oder dass mindestens ein zusätzlicher Inhaltsstoff mit einem pH-Wert bei oder unter dem pH-Wert des mindestens einen gewünschten Inhaltsstoffs verwendet wird;

oder

Durchführen eines zweiten Inhaltstoffempfehlungsprozesses als Reaktion darauf, dass der erste Indikator anzeigt, dass der Benutzer nicht beabsichtigt, einen sauerstoffreduzierten Lebensmittelverarbeitungsschritt während der Herstellung der Nahrungszubereitung zu verwenden, wobei der zweite Inhaltstoffidentifizierungsprozess umfasst:

Bestimmen, ob der pH-Wert des mindestens einen gewünschten Inhaltsstoffs bei, über oder unter einem zweiten vorbestimmten pH-Wert liegt, wobei der zweite vorbestimmte pH-Wert nicht weniger als 5,5 ist; und
Empfehlen, ob und welche von einem oder mehreren zusätzlichen Inhaltsstoffe verwendet werden sollten durch:

als Reaktion auf die Feststellung, dass der pH-Wert des mindestens einen gewünschten Inhaltsstoffs bei oder unter dem zweiten vorbestimmten pH-Wert liegt, Empfehlen, dass mindestens ein zusätzlicher Inhaltsstoff mit einem pH-Wert über dem zweiten vorbestimmten pH-Wert verwendet wird;

als Reaktion auf die Feststellung, dass der pH-Wert des mindestens einen gewünschten Inhaltsstoffs nicht unter dem zweiten vorbestimmten pH-Wert liegt, Empfehlen, dass kein zusätzlicher Inhaltsstoff verwendet werden muss oder dass mindestens ein zusätzlicher Inhaltsstoff mit einem pH-Wert über dem pH-Wert des mindestens einen gewünschten Inhaltsstoffs verwendet werden kann.

14. Verarbeitungssystem nach Anspruch 13, wobei der erste vorbestimmte pH-Wert nicht größer als 5,0 ist, wobei der erste vorbestimmte pH-Wert vorzugsweise im Bereich von 3,0 bis 5,0 und besonders bevorzugt im Bereich von 3,0 bis 4,0 liegt; und wobei der zweite vorbestimmte pH-Wert nicht weniger als 6,0 beträgt.

## Revendications

1. Procédé mis en œuvre par ordinateur pour recommander si et lequel parmi un ou plusieurs ingrédients supplémentaires devraient être utilisés par un utilisateur dans la fabrication d'une préparation nutritionnelle pour améliorer la rétention de folate pendant la fabrication de la préparation nutritionnelle, le procédé consistant à :

recevoir un premier indicateur qui indique si l'utilisateur a l'intention d'utiliser une procédure de traitement des aliments à teneur réduite en oxygène pour fabriquer la préparation nutritionnelle ;
recevoir un deuxième indicateur qui indique au moins un ingrédient souhaité que l'utilisateur a l'intention d'utiliser pour fabriquer la préparation nutritionnelle, l'au moins un ingrédient souhaité comprenant au moins un ingrédient riche en folate ;
déterminer la valeur du pH de l'au moins un ingrédient souhaité ; et
exécuter un premier processus de recommandation d'ingrédients en réponse au premier indicateur indiquant que l'utilisateur a l'intention d'utiliser une étape de traitement des aliments à teneur réduite en oxygène pendant la fabrication de la préparation nutritionnelle, où le premier processus de recommandation d'ingrédients consiste à :

déterminer si la valeur de pH de l'au moins un ingrédient souhaité est égale, supérieure ou inférieure à une première valeur de pH prédéterminée, où la première valeur de pH prédéterminée n'est pas supérieure à 5,5 ;
recommander si et lequel parmi un ou plusieurs ingrédients supplémentaires devraient être utilisés en :

recommandant, en réponse à la détermination que la valeur de pH de l'au moins un ingrédient souhaité est égale ou supérieure à la première valeur de pH prédéterminée, qu'au moins un ingrédient supplémentaire, ayant une valeur de pH inférieure à la première valeur de pH prédéterminée, soit utilisé ; et
recommandant, en réponse à la détermination que la valeur de pH de l'au moins un ingrédient souhaité est inférieure à la première valeur de pH prédéterminée, soit qu'aucun ingrédient supplémentaire ne doit être utilisé, soit qu'au moins un ingrédient supplémentaire, ayant une valeur de pH égale ou inférieure à la valeur du pH de l'au moins un ingrédient souhaité, soit utilisé ;

ou

exécuter un deuxième processus de recommandation d'ingrédients en réponse au premier indicateur indiquant que l'utilisateur n'a pas l'intention d'utiliser une étape de traitement des aliments à teneur réduite en oxygène pendant la fabrication de la préparation nutritionnelle, le deuxième processus d'identification d'ingrédients consistant à :

déterminer si la valeur de pH de l'au moins un ingrédient souhaité est égale, supérieure ou inférieure à une deuxième valeur de pH prédéterminée, où la deuxième valeur de pH prédéterminée n'est pas moins de 5,5 ; et
recommander si et lequel parmi un ou plusieurs ingrédients supplémentaires devraient être utilisés en :

recommandant, en réponse à la détermination que la valeur de pH de l'au moins un ingrédient souhaité est égale ou inférieure à la deuxième valeur de pH prédéterminée, qu'au moins un ingrédient supplémentaire, ayant une valeur de pH supérieure à la deuxième valeur de pH prédéterminée, soit utilisé ;
recommandant, en réponse à la détermination que la valeur de pH de l'au moins un ingrédient souhaité n'est pas inférieure à la deuxième valeur de pH prédéterminée, qu'aucun ingrédient supplémentaire

20

ne doit être utilisé ou qu'au moins un ingrédient supplémentaire, ayant une valeur de pH supérieure à la valeur de pH de l'au moins un ingrédient souhaité, peut être utilisé.

2. Procédé selon la revendication 1, dans lequel la première valeur de pH prédéterminée n'est pas supérieure à 5,0, de préférence dans la plage de 3,0 à 5,0, et plus préférablement dans la plage de 3,0 à 4,0.

3. Procédé selon l'une quelconque des revendications 1 ou 2, dans lequel l'étape de traitement des aliments à teneur réduite en oxygène est une étape de mélange, de réduction en purée, de broyage ou d'extraction de jus à teneur réduite en oxygène.

4. Procédé selon l'une quelconque des revendications 1 à 3, dans lequel, pendant le premier processus de recommandation d'ingrédients, l'étape consistant à recommander si et lequel parmi un ou plusieurs ingrédients supplémentaires devraient être utilisés comprend la recommandation d'un groupe d'un ou plusieurs ingrédients, y compris l'au moins un ingrédient souhaité, qui ont ensemble une valeur de pH inférieure à la première valeur de pH prédéterminée.

5. Procédé selon la revendication 4, dans lequel, pendant le premier processus de recommandation d'ingrédients, l'étape de recommander si et lequel parmi un ou plusieurs ingrédients supplémentaires devraient être utilisés comprend l'identification d'une recette définissant le groupe d'un ou plusieurs ingrédients.

6. Procédé selon l'une quelconque des revendications 1 à 5, dans lequel, pendant le premier processus de recommandation d'ingrédients, l'étape consistant à recommander si et lequel parmi un ou plusieurs ingrédients supplémentaires devraient être utilisés consiste à:

   fournir, en réponse à la détermination que la valeur de pH de l'au moins un ingrédient souhaité est égale ou supérieure à la première valeur de pH prédéterminée, une sortie perceptible par l'utilisateur énumérant une pluralité d'ingrédients supplémentaires différents ayant une valeur de pH inférieure à la première valeur de pH prédéterminée, où de préférence la liste est ordonnée de la valeur de pH la plus basse à la valeur de pH la plus élevée; et/ou
   fournir, en réponse à la détermination que la valeur de pH de l'au moins un ingrédient souhaité est inférieure à la première valeur de pH prédéterminée, une sortie perceptible par l'utilisateur énumérant une pluralité d'ingrédients supplémentaires différents ayant une valeur de pH inférieure à la valeur de pH de l'au moins un ingrédient souhaité, où de préférence la liste est ordonnée de la valeur de pH la plus basse à la valeur de pH la plus élevée.

7. Procédé selon la revendication 6, dans lequel le deuxième indicateur indique en outre un poids de chacun au moins un ingrédient souhaité et le procédé comprend en outre, après avoir exécuté le premier processus de recommandation d'ingrédients:

   la réception d'un troisième indicateur indiquant au moins un ingrédient supplémentaire sélectionné, un ingrédient supplémentaire sélectionné étant l'un de la pluralité d'ingrédients supplémentaires potentiels différents qui a été sélectionné par un utilisateur; et
   la recommandation d'un poids minimum pour chaque ingrédient supplémentaire sélectionné de sorte que la combinaison de l'au moins un ingrédient souhaité et d'au moins un ingrédient supplémentaire sélectionné soit inférieure à la première valeur de pH prédéterminée.

8. Procédé selon l'une quelconque des revendications 1 à 7, dans lequel le deuxième indicateur indique en outre un poids de chacun au moins un ingrédient souhaité et le procédé comprend en outre, après avoir effectué le premier processus de recommandation d'ingrédient et si au moins un ingrédient supplémentaire est recommandé, la recommandation d'un poids minimum pour chaque au moins un ingrédient supplémentaire de sorte que la combinaison de l'au moins un ingrédient souhaité et de l'au moins un ingrédient supplémentaire soit inférieure à la première valeur de pH prédéterminée.

9. Procédé selon la revendication 1, dans lequel la deuxième valeur de pH prédéterminée n'est pas moins de 6,0, et plus préférablement pas moins de 6,6.

10. Procédé selon l'une quelconque des revendications 1 ou 9, dans lequel, pendant le deuxième processus de recommandation d'ingrédients, l'étape consistant à recommander si et lequel parmi un ou plusieurs ingrédients supplémentaires devraient être utilisés consiste à:

fournir, en réponse à la détermination que la valeur de pH de l'au moins un ingrédient souhaité est égale ou inférieure à la deuxième valeur de pH prédéterminée, une sortie perceptible par l'utilisateur énumérant une pluralité d'ingrédients supplémentaires différents ayant une valeur de pH supérieure à la deuxième valeur de pH prédéterminée, où de préférence la liste est ordonnée de la valeur de pH la plus élevée à la valeur de pH la plus basse.

11. Procédé selon la revendication 10, dans lequel le deuxième indicateur indique en outre un poids de chacun au moins un ingrédient souhaité et le procédé comprend en outre, après avoir exécuté un deuxième processus de recommandation d'ingrédients dans lequel une sortie perceptible par l'utilisateur est fournie:

la réception d'un quatrième indicateur indiquant au moins un ingrédient supplémentaire sélectionné, un ingrédient supplémentaire sélectionné étant l'un de la pluralité d'ingrédients supplémentaires potentiels différents qui a été sélectionné par un utilisateur; et
la recommandation d'un poids minimum pour chaque ingrédient supplémentaire sélectionné de sorte que la combinaison de l'au moins un ingrédient souhaité et d'au moins un ingrédient supplémentaire sélectionné soit supérieure à la deuxième valeur de pH prédéterminée.

12. Programme d'ordinateur comprenant des moyens de codage pour mettre en œuvre le procédé selon l'une quelconque des revendications 1 à 11 lorsque ledit programme est exécuté sur un ordinateur.

13. Système de traitement pour déterminer si des ingrédients supplémentaires doivent être utilisés par un utilisateur pour assurer au moins une quantité prédéterminée de rétention de folate dans la fabrication d'une préparation nutritionnelle, le système de traitement étant adapté pour:

recevoir un premier indicateur qui indique si l'utilisateur a l'intention d'utiliser une étape de transformation des aliments à teneur réduite en oxygène pendant la fabrication de la préparation nutritionnelle;
recevoir un deuxième indicateur qui indique au moins un ingrédient souhaité que l'utilisateur a l'intention d'utiliser dans la fabrication de la préparation nutritionnelle;
déterminer la valeur du pH de l'au moins un ingrédient souhaité; et
exécuter un premier processus de recommandation d'ingrédients en réponse au premier indicateur indiquant que l'utilisateur a l'intention d'utiliser une étape de traitement alimentaire à teneur réduite en oxygène pendant la fabrication de la préparation nutritionnelle, le premier processus de recommandation d'ingrédients consistant à:

déterminer si la valeur de pH de l'au moins un ingrédient souhaité est égale, supérieure ou inférieure à une première valeur de pH prédéterminée, où la première valeur de pH prédéterminée n'est pas supérieure à 5,5;
recommander si et lequel parmi un ou plusieurs ingrédients supplémentaires devraient être utilisés en:

recommandant, en réponse à la détermination que la valeur de pH de l'au moins un ingrédient souhaité est égale ou supérieure à la première valeur de pH prédéterminée, qu'au moins un ingrédient supplémentaire, ayant une valeur de pH inférieure à la première valeur de pH prédéterminée, soit utilisé; et
recommandant, en réponse à la détermination que la valeur de pH de l'au moins un ingrédient souhaité est inférieure à la première valeur de pH prédéterminée, soit qu'aucun ingrédient supplémentaire ne doit être utilisé, soit qu'au moins un ingrédient supplémentaire, ayant une valeur de pH égale ou inférieure à la valeur du pH de l'au moins un ingrédient souhaité, soit utilisé;

ou

exécuter un deuxième processus de recommandation d'ingrédients en réponse au premier indicateur indiquant que l'utilisateur n'a pas l'intention d'utiliser une étape de traitement des aliments à teneur réduite en oxygène pendant la fabrication de la préparation nutritionnelle, le deuxième processus d'identification d'ingrédients consistant à:

déterminer si la valeur de pH de l'au moins un ingrédient souhaité est égale, supérieure ou inférieure à une deuxième valeur de pH prédéterminée, où la deuxième valeur de pH prédéterminée n'est pas moins de 5,5; et
recommander si et lequel parmi un ou plusieurs ingrédients supplémentaires devraient être utilisés en:

recommandant, en réponse à la détermination que la valeur de pH de l'au moins un ingrédient souhaité est égale ou inférieure à la deuxième valeur de pH prédéterminée, qu'au moins un ingrédient supplémentaire, ayant une valeur de pH supérieure à la deuxième valeur de pH prédéterminée, soit utilisé; recommandant, en réponse à la détermination que la valeur de pH de l'au moins un ingrédient souhaité n'est pas inférieure à la deuxième valeur de pH prédéterminée, qu'aucun ingrédient supplémentaire ne doit être utilisé ou qu'au moins un ingrédient supplémentaire, ayant une valeur de pH supérieure à la valeur de pH de l'au moins un ingrédient souhaité, peut être utilisé.

14. Système de traitement selon la revendication 13, dans lequel la première valeur de pH prédéterminée n'est pas supérieure à 5,0, dans lequel la première valeur de pH prédéterminée est de préférence dans la plage de 3,0 à 5,0, et plus préférablement dans la plage de 3,0 à 4,0; et dans lequel la deuxième valeur de pH prédéterminée n'est pas moins de 6,0.

FIG. 1

FIG. 2

FIG. 3

FIG. 4

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **N. DELCHIER et al.** *Food chemistry,* 2014, vol. 165, 85-91